# EUROPEAN PATENT SPECIFICATION

(11) **EP 1 270 033 B1**
(45) Date of publication and mention of the grant of the patent: **05.01.2005**
(21) Application number: 02702415.7
(22) Date of filing: 22.02.2002
(51) Int. Cl.: A61M 5/175, A61M 39/00

(54) **DEVICE FOR CONTROLLING THE FLOW VOLUME OF MEDICATION PRODUCTS**
VORRICHTUNG ZUM KONTROLLIEREN DES STROMUNGSVOLUMENS VON MEDIZINISCHES PRODUKTEN
DISPOSITIF DE CONTROLE DU DEBIT POUR PRODUITS MEDICAMENTEUX

(30) Priority: 28.02.2001 ES 200100472
(43) Date of publication of application: 02.01.2003
(73) Proprietor: Grifols Lucas, Victor, E-08150 Parets del Valles (Barcelona) (ES)
(72) Inventor: Grifols Lucas, Victor, E-08150 Parets del Valles (Barcelona) (ES)
(74) Representative: Duran Moya, Luis-Alfonso
(86) International application number: PCT/ES2002/000078
(87) International publication number: WO 2002/068021

(56) References cited:
- ES-A6- 2 028 740

## Description

The present invention is intended to disclose a device for controlling the flow of medicinal products, which has appreciable features of novelty and inventive step.

The device of the present invention is intended for the precise control of medicinal products such as solutions for infusion into the body of patients.

Conventionally, various devices have been used for the intentional reduction of the passage of the tubes supplying medicinal products for infusion into the patient's venous system through a catheter. These known systems suffer from lack of precision in the adjustment of the through-flow and improbable repeatability since the effective flow depends on the precise position of the closure device, which cannot be controlled accurately.

A considerable improvement was introduced by Spanish patent No. 9100691, relating to a flow regulator for catheters and the like in which a gauged duct produced by heating and compression of a tube of thermoplastic material, with the simultaneous introduction of a fine calibrated rod in an axial arrangement inside the tube, was disclosed.

However, owing to its particular configuration, this device permits solely the passage of a fixed and predetermined flow so that, in a few cases, there may be some difficulties when the medicinal substances administered to the patient are liable to cause side effects since, since the whole dose is administered by means of the appropriate gauging device, in the event of adverse side effects appearing in the patient, the interruption of the introduction of the medicinal substance will take place when a considerable quantity of the substance has already been administered to the patient.

To overcome this disadvantage, the inventors have devised the device of the present invention, which achieves very easy positioning and handling characteristics of a flow-control device and at the same time also enables a pilot supply with a greatly reduced flow to be produced in the initial phase, allowing the possible appearance of adverse side-effects in the patient to be observed prior to the administration of the medicinal substance in the total quantity envisaged for the treatment.

To achieve its object, the present invention provides for the production of a gauging device having two or more channels in parallel, each of which carries an individual gauging element, one of these being a pilot gauging element and the others being those which may be necessary for the supply of the definitive flow, so that the pilot gauging element can provide a reduced quantity of the medicinal substance to the patient for the purposes of testing for the possible appearance of adverse reactions and the other gauging elements will provide the total flow necessary for the treatment of the patient. The two or more tubes carrying the gauging elements are joined at their ends, forming a single inlet and a single outlet. The production of the flaw-control device in this form enables it to be mounted in the supply line from the container carrying the substance to be infused to the tube connected to the patient's catheter, the gauging elements being closed by conventional clamping or closure of their supply tube by means of a breakable valve, and the supply to the catheter taking place solely with the pilot tube. After a precautionary period of time, if no adverse reactions appear in the patient, the supply tube of the necessary gauging element or elements will be opened so that, from that moment, the liquid will be administered to the patient in accordance with the total flow necessary for the treatment. If a larger number of tubes is provided, they may be opened progressively.

For a better understanding, some drawings of a flow-control device formed in accordance with the present invention are appended by way of non-limiting example.
Figure 1 is a view of the device as a whole.
Figures 2,3 and 4 are respective sections through one of the gauging elements, taken in the planes indicated.

As shown in the drawings, the device of the present invention comprises two or more flexible tubes, for example, two tubes 1 and 2, each of which has a precision gauging element 3 or 4, for example, of the type disclosed in Utility model No. 9100691; the tubes 1 and 2 are joined, at one end, by means of a Y-shaped connector 5, to a product-inlet tube 6 provided with a luer connector 7 or the like whereas, at the other end, they are joined, by means of a further Y-type connector 8, to a second, outlet tube 9 and to the connector 10 for the catheter-supply tube.

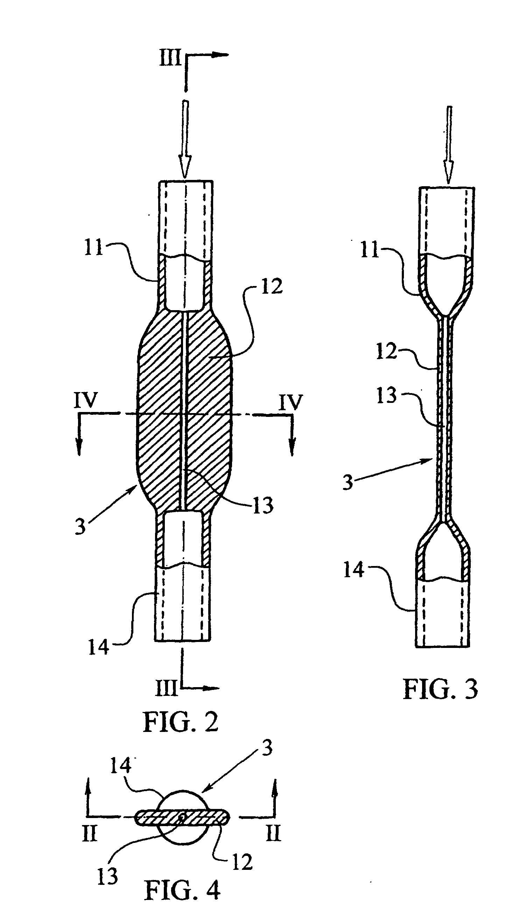

As shown in Figures 2 to 4, the individual gauging elements comprise, substantially, a tubular element 11 in which an intermediate flattened portion 12 is formed; a fine precision duct 13 is formed therein and puts the inlet tube 11 into communication with the outlet tube 14, controlling the flow precisely.

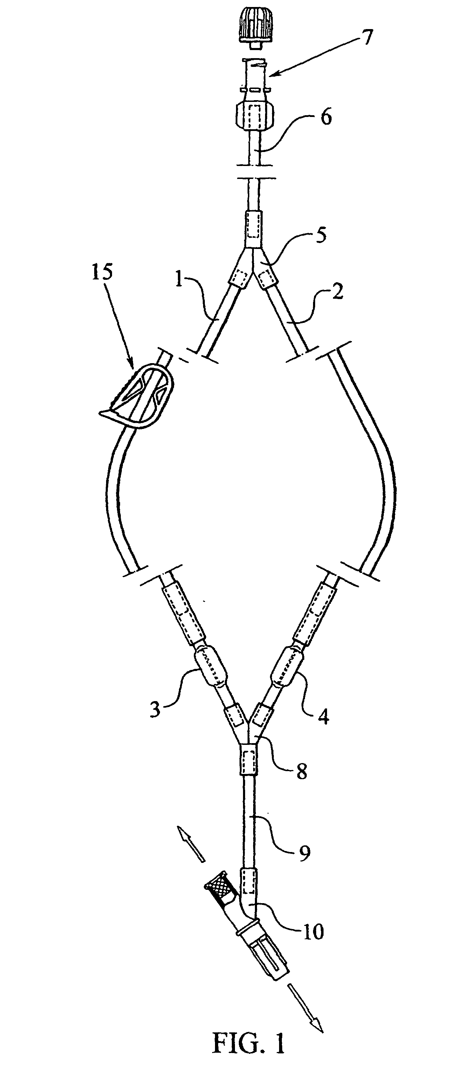

One of the two or more gauging elements 3 and 4, for example, that indicated 4, will have a much smaller flow than the other gauging elements, for example, the gauging element 3, that is, a "pilot" flow, so that, when the device is in use, the main gauging element will be closed by means of a conventional clamping device or breakable valve, such as that indicated 15 in Figure 1, and the administration of the medicinal substance will therefore take place solely through the smaller-capacity gauging element 4 in a quantity which is referred to as a "pilot" quantity, in the sense that it enables any adverse reactions of the patient to be observed prior to the administration of the medicinal substance, without risk to the patient, owing to the small quantity of product administered. After the possible appearance of adverse reactions has been observed, as indicated, after a precautionary administration period, for example, of half an hour, if there are no adverse reactions, the tube 1 for the larger-capacity gauging element or elements 3 etc. will be opened, and from that moment, the outlet tube 9 will be supplied through the two or more gauging elements 3 and 4 which will jointly provide the quantity of medicinal substance necessary for the treatment. As is clear, the formal classification of the treatment flow of the flow-control device will take account of the liquid-flow characteristics which are possible owing to the operation of the gauging elements 3 and 4 in parallel.

A "vent" filter may optionally be inserted in the outlet tube 9 for purging air from the tubes.

The different flow-gauging elements or regulators may be colour-coded according to their diameter to facilitate their identification, particularly for home treatments.

Although it is not shown in the drawings, the tube 2 may also incorporate a closure device such as a clamp, a breakable valve, or the like.

## Claims

1. A flow-control device for medicinal products, **characterized in that** it comprises two or more tubes (1,2) associated, in parallel, with a common inlet and a common outlet for their connection to the supply line of a substance to be infused, each of the said tubes having a precision gauging element (3, 4), one of which has a much smaller flow than the other or others, respective tube-closure devices (15) being associated with the tube or tubes carrying the larger-capacity gauging elements, to permit optional opening or closure thereof.

2. A flow-control device for medicinal products according to Claim 1, **characterized in that** the gauging elements of the tubes in parallel are of the flexible-tube type with a flattened region (12) and a precision axial orifice (13).

## Patentansprüche

1. Ein Strömungswächter für medizinische Gegenstände, **dadurch gekennzeichnet, daß** es zwei Röhren (1, 2) umfaßt, die parallel zueinander mit einem gemeinsamen Einlaß und einem gemeinsamen Auslaß verbunden sind zu ihrer Anbindung an eine Versorgungsleitung einer zu infundierenden Substanz, wobei jede der Röhren ein Präzisionsmeßelement (3, 4) besitzt, von denen eines einen viel kleineren Durchfluß besitzt als das oder die anderen, wobei Röhrenschließeinrichtungen (15) der Röhre oder den Röhren, die die Meßelemente mit größerer Kapazität tragen, jeweils zugeordnet sind, um deren optionales Öffnen oder Schließen zu ermöglichen.

2. Ein Strömungswächter für medizinische Gegenstände gemäß Anspruch 1, **dadurch gekennzeichnet, daß** die Meßelemente der parallelen Röhren aus einem flexiblen Rohrtyp bestehen mit einem flach ausgebildeten Bereich (12) und einer Präzisionsaxialöffnung (13).

## Revendications

1. Dispositif de contrôle de débit pour produits médicinaux,
**caractérisé en ce qu'**il comprend deux, ou plus de deux, tubes (1,2) associés en parallèle, avec une entrée commune et une sortie commune pour leur connexion à la ligne d'alimentation d'une substance à perfuser, chacun desdits tubes ayant un élément de dosage de précision (3,4) dont l'un a un débit beaucoup plus petit que l'autre ou que les autres, des dispositifs de fermeture de tube respectifs (15) étant associés au tube ou aux tubes portant les éléments de dosage de plus grande capacité, pour permettre leur ouverture ou fermeture facultatives.

2. Dispositif de contrôle de débit pour produits médicinaux selon la revendication 1,
**caractérisé en ce que** les éléments de dosage des tubes en parallèle sont du type à tube souple avec une région aplatie (12) et un orifice axial de précision (13).
